# EUROPEAN PATENT SPECIFICATION

(11) **EP 0 374 940 B1**
(45) Date of publication and mention of the grant of the patent: **15.12.1993**
(21) Application number: 89123744.8
(22) Date of filing: 22.12.1989
(51) Int. Cl.: C07K 7/10, A61K 37/02, A01N 1/00

(54) **Polypeptides isolated from the venom of the spider hololena curta**
Polypeptide, isoliert aus dem Gift von Hololena curta
Polypeptides isolés à partir du venin de hololena curta

(30) Priority: 23.12.1988 US 289175; 29.11.1989 US 439582
(43) Date of publication of application: 27.06.1990
(73) Proprietor: MERRELL DOW PHARMACEUTICALS INC., Cincinnati Ohio 45215-6300 (US)
(72) Inventor: Stapleton, Andrew G., Martinez California 94553 (US)
(74) Representative: VOSSIUS & PARTNER

(56) References cited:
- PROC. NATL. ACAD. SCI. USA, vol. 84, May 1987, pages 3506-3510; C.W. BOWERS et al.: "Identification and purification of an irreversible presynaptic neurotoxin from the venom of the spider Hololena curta"
- THE JOURNAL OF BIOLOGICAL CHEMISTRY, vol. 264, no. 4, 5th February 1989, The American Society for Biochemistry and Molecular Biology, Inc., US; W.S. SKINNER et al.: "Purification and characterization of two classes of neurotoxins from the funnel web spider, Agelenopsis aperta"

## Description

In recent years, scientists have sought to isolate and identify new chemical components from spider venom for use in medicine and agriculture. An overview of recent developments in this field is presented in a publication by H. Jackson and P.N.R. Usherwood, "Spider toxins as tools for dissecting elements of excitatory amino acid transmission", TINS, 11, No. 6, 278-283, (1988). It is reported therein that the toxin of the spider Hololena curta has been investigated and that the component of this spider's venom which is active as an irreversible glutamate antagonist resides in a isolated fraction of the venom of 5,000 to 10,000 Da. The suggestion has been made that this toxin "may represent a class of relatively large, irreversible glutamate antagonists" (Id at 282).

Additionally, Bowers, et al., Proc Natl. Acad. Sci., USA, 84, 3506-3510 (1987), discloses that a fraction isolated from H. curta toxin which contains two subunits of Mᵣ 7000 and 9000, joined by disulfide bonds, where the estimated Mᵣ of the toxin assumedly composed of one of each putative subunit is 16,000, is a potent and long-lasting inhibitor of voltage-dependent presynaptic calcium channels.

Such compounds which affect neuromuscular transmitters are of interest in research and for the treatment of a number of neurologic conditions including epilepsy, hypoxic-ischemic neuronal death, and Huntington's disease (Jackson, et al., "Effects of Spider Venoms on Transmission Mediated by Non-N-Methyl-D-Aspartate Receptors in the Avian Cochlear Nucleus", Excitatory Amino Acid Transmission, 51-54, (1987)), Alan R. Liss, Inc., and Alzheimer's disease as well as for use as natural insecticides.

This invention relates to polypeptides as isolated from the venom of the spider Hololena curta or synthetically produced, consisting of a 38 amino acid sequence of about 4,000 Da, essentially of the formula 
N-Ter-Ala-Asp-Cys-Val-Gly-Asp-Gly-Gln-X-Cys-Ala-Asp-Trp-Y-
Gly-Pro-Tyr-Cys-Cys-Ser-Gly-Tyr-Tyr-Cys-Ser-Cys-Arg-Ser-
Met-Pro-Tyr-Cys-Arg-Cys-Arg-Ser-Asp-Ser

(Formula I)
where X is Arg or Lys; and Y is Ala or Phe, and when X is Arg then Y must be Ala, and when X is Lys then Y must be Phe, and of the formula 
N-Ter-Ser-Cys-Val-Gly-Gln-Tyr-Gly-Arg-Cys-Arg-Ser-Ala-Tyr-
Gln-Asp-Cys-Cys-Asp-Gly-Tyr-Tyr-Cys-Asn-Cys-Ser-Gln-Pro-
Pro-Tyr-Cys-Leu-Cys-Arg-Asn-Asn-Asn
The invention also relates to salts thereof, and to the use of the polypeptides and compositions containing the same as insecticides, and to other applications in which glutamate antagonist activity or calcium antagonist activity is desirable, such as in methods for the treatment of a number of neurologic conditions, including, for example, epilepsy, hypoxic-ischemic neuronal death, and Huntington's disease.

The terms used in this specification, most of which are well known and are commonly used in the art, are employed to describe the following:
Ala--alanine
Arg--arginine
Asn--asparagine
Asp--aspartic acid
Cys--cysteine
Gln--glutamine
Gly--glycine
Leu--Leucine
Lys--lysine
Met--methionine
Phe--phenylalanine
Pro--proline
Ser--serine
Tyr--tyrosine
Val--valine
N-Ter--N terminal amino acid residue

The scope of this invention includes three, sequence-related polypeptides, each isolated from the venom of the spider Hololena curta, or produced synthetically. Each polypeptide of Formula I consists of a 38 amino acid sequence, the amino acid sequence differing between the two polypeptides only at positions 9 (X in Formula I) and 14 (Y in Formula I). The compound wherein X is Arg and Y is Ala has a molecular weight of 4188.9 Da, and the compound wherein X is Lys and Y is Phe has a molecular weight of 4237.4 Da. These molecules have a high content of Cys (8), Ser (5), Tyr (4), basic residues as Arg/Lys (4), Asp (4), and an absence of histidine, threonine, isoleucine and leucine. The molecules also have two dipeptide sequences ¹⁸Cys-¹⁹Cys and ²²Tyr-²³Tyr.

The polypeptide of Formula II consists of a 36 amino acid sequence which has a molecular weight of 4103.0 Da. This molecule has a high content of Cys (8) and Tyr (5), and an absence of histidine, threonine and isoleucine. The molecule also has two dipeptides sequences ¹⁶Cys-¹⁷Cys and ²⁰Tyr-²¹Tyr.

The polypeptide compounds of this invention (hereinafter sometimes referred to as the "polypeptides" or as set forth below as "curtatoxin I, II and III") can be produced in substantially pure form by isolation and purification of select, relatively low molecular weight fractions of H. curta venom. Synthesis of polypeptides can also be accomplished by utilizing recombinant DNA techniques and, additionally, by chemical peptide synthesis processes.

Isolation and purification of the polypeptides of Formulae I and II from relatively low molecular weight fractions of H. curta venom were generally carried out by employing the following, procedures. Whole H. curta venom was initially fractionation by -reverse-phase High-Performance Liquid Chromatography (HPLC), where fractions 33 (X is Arg and Y is Ala, identified as Curtatoxin I), 37 (X is Lys and Y is Phe, identified as Curtatoxin II) and 27 (identified as Curtatoxin III) identified as being of interest. Each fraction was then further purified through anion exchange chromatography and gel-filtration. Final purification was by reverse-phase HPLC which yielded a single symmetrical peak for each fraction, indicative of a homogenous protein component. Specific procedures for isolation and purification of the compounds are presented in the examples.

Peptide synthesis of the compounds of this invention can generally be achieved by the following procedure.

The compounds of this invention are prepared by routine methods for peptide synthesis. It is possible, during the synthesis of certain of the compounds of this invention, that partial racemization can occur. However, the extent of racemization, should such occur, is not sufficient to significantly alter the activity of the compounds of this invention.

The compounds of this invention can be synthesized by classical solution phase synthesis.

Preparation involves the coupling of amino acids or peptide fragments by reaction of the carboxyl function of one with the amino function of another to produce an amide linkage. In order to effectively achieve coupling, it is desirable, first, that all reactive functionalities not participating directly in the reaction be inactivated by the use of appropriate blocking groups, and, secondly, that the carboxyl function which is to be coupled by appropriately activated to permit coupling to proceed. All of this involves a careful selection of both reaction sequence and reaction conditions as well as utilization of specific blocking groups so that the desired peptide product will be realized. Each of the amino acids which is employed to produce the compounds of this invention and which has the particularly selected protecting groups and/or activating functionalities is prepared by techniques well recognized in the peptide art.

Selected combinations of blocking groups are employed at each point of the total synthesis of the compounds of this invention. These particular combinations have been found to function most smoothly. Other combinations would operate in the synthesis of the compounds in this invention, although, perhaps, with a lesser degree of success. Thus, for example, benzyloxycarbonyl, t-butyloxycarbonyl, t-amyloxycarbonyl, p-methoxybenzyloxycarbonyl, adamantyloxycarbonyl, and isobornyloxycarbonyl can be variously employed as amino blocking groups in the synthesis of the compounds of this invention. Furthermore, benzyl (Bzl) generally is employed as the hydroxy-protecting group for the tyrosyl residue even though others, such as p-nitrobenzyl (PNB), p-methoxybenzyl (PMB), and the like, could well be employed.

The carboxyl blocking groups used in preparing the compounds of this invention can be any of the typical ester-forming groups, including, for example, methyl, ethyl, benzyl, p-nitrobenzyl, p-methoxybenzyl, 2,2,2-trichloroethyl, and the like.

Coupling of the suitably protected N-blocked amino acid or peptide fragment with a suitably protected carboxy-blocked amino acid or peptide fragment in preparation of the compounds of this invention consists of rendering the free carboxyl function of the amino acid or peptide fragment active to the coupling reaction. This can be accomplished using any of several well recognized techniques. One such activation technique involves conversion of the carboxyl function to a mixed anhydride. The free carboxyl function is activated by reaction with another acid, typically a derivative of carbonic acid, such as an acid chloride thereof. Examples of acid chlorides used to form mixed anhydrides are ethyl chloroformate, phenyl chloroformate, sec-butyl chloroformate, isobutyl chloroformate, pivaloyl chloride, and the like. Preferably, isobutyl chloroformate is employed.

Another method of activating the carboxyl function for the purpose of carrying out the coupling reaction is by conversion to its active ester derivatives. such active esters include, for example, a 2,4,5-trichlorophenyl ester, a pentachlorophenyl ester, a p-nitrophenyl ester, and the like. Another coupling method available for use is the well-recognized azide coupling method.

The preferred coupling method in preparation of the compounds of this invention involves the use of N,N′-dicyclohexylcarbodiimide (DCC) to activate the free carboxyl function thereby permitting coupling to proceed. This activation and coupling technique is carried out employing an equimolar quantity of DCC relative to the amino acid or peptide fragment and is carried out in the presence of an equimolar quantity of 1-hydroxybenzotriazole (HOBt). The presence of HOBt suppresses undesirable side reactions including the possibility of racemization.

Cleavage of selected blocking groups is necessary at particular points in the synthetic sequence employed in preparation of the compounds of this invention. A chemist of ordinary skill in the art of peptide synthesis can readily select from representative protecting groups those groups which are compatible in the sense that selective cleavage of the product can be accomplished permitting removal of one or more but less than all of the protecting groups present on the amino acid or peptide fragment. These techniques are well recognized in the peptide art. A more complete discussion of the techniques which are available for selective cleavage is provided in the literature in Schroder and Lubke, The Peptides, I, Academic Press, New York, (1965), and especially in the Table provided at pages 72-75 thereof.

Cleavage of carboxyl protecting groups can be accomplished by alkaline saponification. Relatively strong alkaline conditions, typically using an alkali metal hydroxide, such as sodium hydroxide, potassium hydroxide, lithium hydroxide, and the like, are generally employed to deesterify the protected carboxyl, The reaction conditions under which saponification is accomplished are well recognized in the art. Many of the carboxyl blocking groups also can be removed by catalytic hydrogenolysis including, for example, hydrogenolysis in the presence of a catalyst such as palladium on carbon. Furthermore, in those instances in which the carboxyl blocking group is p-nitrobenzyl or 2,2,2-trichloroethyl, deblocking can be accomplished by reduction in the presence of zinc and hydrochloric acid.

Many of the amino blocking groups are cleaved by treating the protected amino acid or peptide with an acid such as formic acid, trifluoroacetic acid (TFA), p-toluenesulfonic acid (TSA), benzenesulfonic acid (BSA), naphthalenesulfonic acid, and the like, to form the respective acid addition salt product. Cleavage of others can be accomplished by treating the blocked amino acid or peptide with a mixture of HBr and acetic acid to produce the corresponding hydrobromide acid addition salt. The particular method or reagent which is employed will depend upon the chemical or physical characteristics of the materials involved in the specific deblocking reaction. The resulting acid addition salt can be converted to a more pharmaceutically acceptable form by treatment with a suitable ion exchange resin, such as DEAE Sephadex A25, Amberlyst A27, and the like.

The hydroxy-protecting group can be retained on the peptide throughout the sequence of its preparation, being removed during the final synthetic step in conjunction with cleavage of the amino blocking group. However, depending upon the conditions employed for removal of the carboxyl blocking group, it may be removed earlier in the preparative sequence. When the carboxyl group is cleaved by alkaline saponification, the hydroxy-protecting group is retained; however, when catalytic hydrogenolysis is employed for removal of the carboxyl protecting group, the hydroxy protecting group also is cleaved. the latter situation does not represent a serious problem since preparation of the compounds of this invention can be accomplished in the presence of an unprotected tyrosyl residue.

Other sequences, of course, are available. One involves coupling a separately prepared N-terminal tripeptide with a separately prepared C-terminal phenylalanine derivative followed by appropriate deblocking of any remaining blocked moieties. Another solution method which can be employed involves the step-wise, sequential addition of single amino acids in construction of the peptide chain beginning with the C-terminal moiety. Reaction techniques such as those described above are employed in this as well as any other contemplated preparative sequence.

Additionally, the compounds of this invention can be produced by recombinant DNA synthesis.

Generally, the complete primary structures of the compounds of this invention were determined by the following procedure. Curtatoxins I, II and III were pyridylethylated according to the procedure of Firedman, et al., J. Biol. Chem., 245, 3868 (1970), as modified by D. Hawke and P. Yuan, in Applied Biosystems Incorporated User Bulletin, No. 28 (1987). The pyridylethylated toxins (PESTS) were then desalted by microbore reverse-phase HPLC, and, after elution approximately 40 minutes into the gradient program, standard automated Edman degradations were performed. The phenylthiohydantion-derivatized amino acids were analyzed at each cycle on a PTH-analyzer directly on-line with a gas-phase sequencer. The peptides were hydrolized by gas-phase hydrolysis in autosampler tubes which were then transferred to an aminoquant analyzer for the detection of primary and secondary amino acids. Initially, several nanomoles of the native toxins were analyzed and each gave high yields of PTH-amino acids resulting in unambiguous assignments for the first 35-37 residues. Cysteines were determined by sequence analysis of the PEST proteins. In order to verify the carboxy-terminal sequence, the PEST form of each peptide was degraded with CNBr to cleave methionine at position 29. The carboxy-terminal fragment was purified by microbore C-18 reverse-phase HPLC and sequenced. The amino acid composition of the hydrolyzed peptide agreed with that composition calculated from the amino acid sequence of the fragment confirming the carboxy-terminal serine residue. Confirmation of the structure was obtained by FAB-MS analysis. The respective MH+ ion molecular weights for Curtatoxin I and II were 4188.7 Da and 4237.4 Da versus the calculated values of 4189.6 Da and 4237.7 Da. The molecular weight for Curtatoxin III was 4103.0 Da which was consistent with that calculated from the molecular weight of the amino acid sequence of the carboxyamidated polypeptide. Specific procedures for determining the primary structures of the compounds of this invention are presented in the Examples below.

The following specific examples are presented to illustrate the process for acquiring and characterizing the compounds of this invention, but they should not be construed as limiting the scope of this invention in any way.

### Example 1

### Purification Protocol for Curtatoxins I and II and III

Hololena curta venom was stored frozen and was chromatographed without an initial freeze-drying step, i.e., direct injection of the venom onto the HPLC column.

The bioassay used to monitor the purification of the Curtatoxins was paralysis of the cricket, Acheta domestica. Following chromatography, column fractions were lyophilised overnight using a Savant Speed-Vac and the residue dissolved in a minimum amount of distilled/deionized water (normally 100-200 µl). An injection of 2 µl was made into the thoracic cavity of a cricket, which was subsequently held in a plastic petri-dish. Fractions containing paralytic material usually produced a paralysis within 5 to 10 minutes which was complete after 2 to 3 hours. The crickets were monitored over at least 24 hours (usually 48 hours) to check for reversibility.

Purification: Step 1 - A pharmacia Pep RPC HR 16/10 column was equilibrated overnight with HPLC-grade water containing 0.05% TFA (trifluoroacetic acid). Whole venom (0.5 ml) was applied to the column vial an injection loop at a flow rate of 2.5 ml/min. The column was washed with 40 ml of equilibration solvent at the above flow rate. Venom components were selectively eluted by running a linear gradient to 60% acetonitrile (containing 0.05% TFA) over 100 minutes at a flow rate of 2.5 ml/min. A fraction collector was used to collect 5 ml (2 minute) fractions which were lyophilised and bioassayed as described above. A rapid, essentially immediate paralysis was seen from Fractions 27, 33 and 37. These fractions were taken through subsequent purification steps.

Essentially any preparative reverse-phase (C18) HPLC column should give a similar, if not identical separation. The Pharmacia column was packed with 15 µl porous silica C_{2/18}. The gradient used in this first step was slower, but it produced excellent resolution of the venom components.

Step 2: Curtatoxin I (from Fraction 33) - A Bio-Rad MicroAnalyzer MA7P+ anion-exchange HPLC column (4.6 x 30 mm) was equilibrated with 20mM Tris pH 7.5 at a flow rate of 0.5 ml/min. Under such isocratic conditions, the paralytic toxin from Fraction 33 did not bind to the column but eluted in the void volume. Fractions were collected by hand while monitoring the absorbance at 220 nm. Inactive components were slightly retarded by this protocol or bound to the column producing a significant purification.

The MicroAnalyzer is slightly different from other anion-exchangers in that it consists of a spherical, pellicular (non-porous) polymer (in most HPLC columns, sample-column interactions occur inside the pores of the packing material). Thus the MicroAnalyzer has a relatively low-capacity which in turn means that yields are high and higher flow-rates can be used. Although the recommended flow-rate for this column is 1.5 ml/min, a slower flow rate gave excellent results.

Step 3: Curtatoxin I - The active material from the preceding step was applied to a Bio-Rad BioSil TSK 250 Gel-Permeation column (300 x 7.5 mm) previously equilibrated with 50 mM Na₂S0₄ / 20 mM Na₂HPO₄ pH 6.8 at a flow-rate of 1.0 ml/min. Under these isocratic conditions, the paralytic activity eluted at an elution volume (Ve) of approximately 12 ml. Several minor contaminants were removed by this process.

Step 4: Curtatoxin I - The final step in the purification of Curtatoxin I was another Reverse Phase step performed on the same column as step 1. However, the column was equilibrated with 15% acetonitrile in water/0.05%TFA at a flow-rate of 2.5 ml/min. Following application of the toxin, the column was washed with 12.5 ml of the equilibration solvent at the above flow. Curtatoxin I was eluted by running a gradient to 35% acetonitrile over 85 minutes at 2.5 ml/min. The elution position was at approximately 26% acetonitrile.

The purified toxin was lyophilised and stored frozen prior to being subjected to sequence analysis.

Step 5: Curtatoxin II (Fraction 37 from Step 1) -Fraction 37 was applied to the Gel-Permeation column as described for Step 3 except that the flow rate was 0.5 ml/min. The toxic component eluted at an elution volume (Ve) of approximately 16 ml.

Step 6: Curtatoxin II - The active material from the preceding step was chromatographed exactly as described for Step 4. The active material eluted at a position corresponding to approximately 31% acetonitrile.

Step 7: Curtatoxin III (Fraction 27 from step 1) -Purification of curtatoxin III was accomplished by rechromatography of fraction 27 (reconstituted in 100 µl of 0.1% TFA in H₂O) on an Aquapore RP-300 microbore reverse phase column (2.1 X 30 mm) using an Applied Biosystems Inc. model 130 protein-peptide separation system. The gradient was linear from 100% buffer A (0.1% TFA in H₂O) to 100% buffer B (0.085% TFA and 70% acetonitrile in H₂O) over 150 min at a flow rate of 100 µl/min. The toxin was also purified by microbore reverse phase HPLC after reduction and pyridylethylation with 4-vinyl pyridine.

### Example 2

### Primary structure determination

### Pyridylethylation and Cyanogen Bromide Degradation of Curtatoxins.

Prior to sequence analysis, purified curtatoxins were pyridylethylated according to the procedure of Friedman et al., (1970) as modified by Hawke and Yuan (1987) for use with small amounts of protein. Briefly, 10 µg of each curtatoxin were dissolved in 44 µl of 6 M guanidine-HCl, 0.25 M Tris-CHl, pH 8.5 and sequentially reacted with 3 µl of 10% β-mercpatoethanol and 3 µl of 4-vinylpyridine for 2 hours at room temperature. Pyridylethylated toxins were then desalted by microbore reverse-phase HPLC employing an Aquapore RP-300 column (2.1 x 30 mM) on an Applied Biosystems Inc. model 130 protein peptide separation system. The gradient was linear and ranged from 100% Buffer A, (0.1% trifluoroacetic acid in H₂O) to 100% Buffer, B, (.085% trifluoroacetic acid and 70% acetonitrile in H₂O over 150 min at a flow rate of 100 µl/min. The pyridylethylated spider toxins (PESTS) eluted approximately 40 min into the gradient program and 5-10 min earlier than the unmodified proteins.

To produce carboxy-terminal fragments, 10 µg of each PEST I and II was dissolved in 330 µl of 70% formic acid. Several crystals of CNBr were added to the samples which were then flushed with nitrogen, sealed and placed in the dark for 24 hours at room temperature. The samples were dried on a speedvac centrifuge (Savant), redissolved in 50 µl of 0.1% TFA/H₂O and repurified by microbore reverse-phase HPLC. The gradient was linear and ranged from 100% buffer A (0.1% TFA in H₂O) to 100% buffer B (0.085% TFA and 70% acetonitrile in H₂O) over 150 min. Carboxy-terminal fragments of PEST III were generated by tryptic digestion of 18 µg of toxin at an enzyme: substrate weight ratio of 1:50; digests were performed for 20 h at room temperature in 1% ammonium bicarbonate, pH 9.0. Peptides were purified by microbore reverse phase HPLC as described above.

Automated Edman degradations were performed on a model 470A protein-peptide sequencer (Applied Biosystems, Inc.) with reagents, instructions and standard programs supplied by the manufacturer. The phenylthiohydantion-derivatized amino acids were analyzed at each cycle on a model 120 PTH-analyzer (Applied Biosystems, Inc.) directly on-line with the 470A gas-phase sequencer.

Amino Acid Analysis - Amino acid analysis identification was done with a dedicated thermospray liquid chromatography - mass spectrometry (LC-MS) system consisting of a Hewlett Packard 5790 mass selective detector mounted in a Vestec 101 thermospray interface consisting of both the thermospray ion source and associated vacuum system. Acid hydrolysates were reacted with phenylisothiocyanate (PITC) to form the phenylthiocarbamyl (PTC) amino acids prior to LC-MS analysis. Quantitative results were obtained on a Hewlett Packard aminoquant analyzer that employs a double derivatization chemistry, o-phthalaldehyde and 9-fluoroenylmethyl chloroformate, for the determination of primary and secondary amino acids, respectively.

Amino Acid Sequence Analyses - Automated Edman degradations were performed on a Model 470A protein-peptide sequencer (Applied Biosystems, Inc.) with reagents, instructions and standard programs supplied by the manufacturer. The reagents, instructions and standard programs supplied by the manufacturer. The phenylthiohydantoin-derivatized amino acids were analyzed at each cycle on a model 120 PTH-analyzer (Applied Biosystems, Inc.) directly on-line with the 470A gas-phase sequencer.

FAB Mass Spectrometry - Molecular weight determinations were performed using a ZAB2-SE double focusing mass spectrometer (VG Analytical, Ltd.). FAB analysis of the intact curtatoxins was accomplished using 1 µg of the appropriate toxin in a matrix consisting of dithiothreitol, dithioerythritol, and thioglycerol (5:1:6 by wt.) with 1% TFA. Ionization was obtained using a linear scan of the acceleration potential at constant magnetic field strength. The interval was selected to include 2 CsI reference ions whose mass values (4030.0541 and 4289.8640) bracketed the molecular weight region of the curtatoxins. Data were acquired at a mass resolution of 1000 using the MCA mode of the data system. This mode collects consecutive scans of continuum data and integrates them into the same data file. Using this scheme, 3 scans from the peptide were integrated with 3 subsequent scans from the CsI reference. The mass of the toxin was then determined through linear interpolation.

The mass spectra acquired for the carboxy-terminus-containing peptide fragments were obtained using conventional magnet current scans. A mass resolution of 2000 was employed to enable the individual isotopes of the peptides to be resolved. Prior to analysis, mass calibration was obtained using CsI as the reference. Subsequent analyses were performed in a matrix consisting of 1:1 glycerol/thioglycerol (w/w) with 1% TFA using a 17 keV cesium ion beam.

The compounds of this invention are useful as insecticides. Typically, the polypeptides can be administered in amounts of at least about 3 micrograms per gram of body weight to produce some insecticidal activity. Preferably, in amounts of at least about 9 micrograms per gram of body weight is employed. The insecticidal properties of the compounds of this invention can readily be determined by standard and well-known procedures. The following procedure demonstrates insecticidal activity of the polypeptides.

Crickets, of the genus/species Acheta domestica, were injected with either whole spider venom, or purified fraction of spider venom, while control crickets were injected with distilled water alone. While Hololena Curta venom (50 mg/ml as measured by the Bradford/Coomassie Blue assay) was mixed with deionized water and 0.5 µl (25 µg protein) was injected into the thoracic cavity of a cricket caused an immediate and irreversible paralysis. Approximately 880 µg of curtatoxin II (fraction 37) were dissolved in 150 µl of deionized water. A dilution of 5.86 µg/µl was used on one test group of 5 crickets (average weight = .384 grams), with 2.93 µg of polypeptide being injected into each cricket. Loss of righting response and paralysis was observed, and all 5 crickets were dead 48 hours post-injection, while all 5 control were alive. A second solution of 1.95 µg/µl was prepared and 0.98 µg of polypeptide was likewise injected into 5 crickets (average weight = .314 g ). Two of the 5 crickets were dead after 48 hours, while all 5 control crickets were normal. Further diluted solutions were tested but did not produce mortality.

The above results demonstrate that potent insecticidal activity was obtained with the compound(s) of this invention.

The compounds of this invention are also useful as glutamate receptor and calcium channel antagonists effective in treating neurological conditions including convulsions, Alzheimers and Huntington diseases, post cerebral ischemia which can follow strokes, and the like.

The antagonist properties of these compounds can readily be determined by standard and well-known procedures, such as those set forth by L. Vyklicky, et al., in Neuroscience Letters, 68, 227-231 (1986), and by C.W. Bowers, et al., in Proc. Natl. Acad. Sci., USA, 84, 3506-3510 (1987).

Typically, the compounds of this invention are administered to a mammal e.g. a human patient in need of treatment, in an amount effective to produce a glutamate receptor or calcium channel antagonist response, thereby resulting in the treatment of the conditions hereinabove noted.

The polypeptide compounds of this invention may be used in free acid form or as salts. The expression "pharmaceutically-acceptable salt" means any organic or inorganic addition salt of the compounds of Formula I which are relatively non-toxic and innocuous to a patient at concentrations consistent with effective activity so that the side effects ascribable to the salt do not vitiate the beneficial effects of the polypeptides of Formula I. These salts are included within the scope of this invention. Such salts include ammonium salts; alkali metal salts, such as sodium and potassium salts; alkaline earth metal salts, such as the calcium and magnesium salts; salts with organic bases such as, for instance, dicyclohexylamine salts, N-methyl-D-glucamine, and salts with amino acids such as arginine and lysine; and the like. Also salts with organic and inorganic acids can be prepared, such as, for example, those formed with the following acids: hydrochloric, hydrobromic, sulfonic, sulfuric, phosphoric, nitric, ascorbic methanesulfonic, acetic, propionic, tartaric, citric, lactic, malic, mandelic, cinnamic, palmitic, itaconic, fumaric, benzenesulfonic and toluenesulfonic. The non-toxic, physiologically acceptable salts are preferred, although other salts are also useful, for, for example, isolating or purifying the product.

The salts can be formed by conventional means such as by reacting the free acid forms of the product with one or more equivalents of the appropriate base in a solvent or medium in which the salt is insoluble, or in a solvent such as water which is then removed *in vacuo* or by freeze-drying, or by exchanging the cations of an existing salt for another cation on a suitable ion exchange resin.

A patient, for the purpose of treating neurological disorders, is a mammal, including a human, in need of treatment for a particular condition, injury or disease. The amount of active ingredient (i.e., a polypeptide of Formula I or II) to be administered to a patient for the treatment of neurological disorders can vary widely according to such considerations as the particular dosage unit employed, the period of treatment, the age and sex of the patient treated, and the extent of the condition being treated, the polypeptide employed, whether concommittant treatment with other drugs is being utilized, and the like.

The polypeptides of this invention can be utilized to achieve the desired pharmacological effect by administration to a patient in need thereof in an appropriately formulated pharmaceutical composition. Therefore, the present invention includes pharmaceutical compositions which comprise a pharmaceutically-acceptable carrier and a pharmaceutically-effective amount of a compound of Formula I. A pharmaceutically-acceptable carrier is any carrier which is relatively non-toxic and innocuous to a patient at concentrations consistent with effective activity of the active ingredient so that any side effects ascribable to the carrier do not vitiate the beneficial effects of the active ingredient. A pharmaceutically-effective amount of compound is that amount which produces a result or exerts an influence on the particular condition being treated. The compounds of Formula I and Formula II can be administered with a pharmaceutically-acceptable carrier using conventional dosage unit forms for oral or parenteral administration.

Physicians will determine the particular dosage of the compounds of this invention which is most suitable. The selected dosages will vary depending upon the factors noted above, but will typically range from about 0.01 to about 10 mg/kg.

The present invention also encompasses compositions containing the polypeptides and an inert carrier. Such compositions can be used for diagnostic applications or as analytical references or standards, as well as for insecticidal applications. Therefore, the present invention includes compositions which comprise an inert carrier and a polypeptide of this invention, or a salt thereof. An inert carrier is any material which does not inter-react with the compound to be carried and which lends support, means of conveyance, bulk, traceable material, and the like to the compound to be carried. An effective amount of the compound is that amount which performs in a desirable manner for there noted uses, and produces a result, or exerts an influence on the particular procedure being performed.

## Claims (Claims for the following Contracting State(s): AT, BE, CH, DE, FR, GB, IT, LI, LU, NL, SE)

1. A polypeptide of the formula 
N-Ter-Ala-Asp-Cys-Val-Gly-Asp-Gly-Gln-X-Cys-Ala-Asp-
Trp-Y-Gly-Pro-Tyr-Cys-Cys-Ser-Gly-Tyr-Tyr-Cys-Ser-Cys-
Arg-Ser-Met-Pro-Tyr-Cys-Arg-Cys-Arg-Ser-Asp-Ser
(Formula I)
 wherein X is Arg or Lys; and Y is Ala or Phe, with the proviso that when X is Arg then Y must be Ala, and when X is Lys then Y must be Phe,
and the salts thereof.

2. A polypeptide according to Claim 1 of the formula

N-Ter-Ala-Asp-Cys-Val-Gly-Asp-Gly-Gln-Arg-Cys-Ala-Asp-
Trp-Ala-Gly-Pro-Tyr-Cys-Cys-Ser-Gly-Tyr-Tyr-Cys-Ser-
Cys-Arg-Ser-Met-Pro-Tyr-Cys-Arg-Cys-Arg-Ser-Asp-Ser.
(Curtatoxin I)

3. A polypeptide according to Claim 1 of the formula

N-Ter-Ala-Asp-Cys-Val-Gly-Asp-Gly-Gln-Lys-Cys-Ala-Asp-
Trp-Phe-Gly-Pro-Tyr-Cys-Cys-Ser-Gly-Tyr-Tyr-Cys-Ser-
Cys-Arg-Ser-Met-Pro-Tyr-Cys-Arg-Cys-Arg-Ser-Asp-Ser.
(Curtatoxin II)

4. A polypeptide of the formula 
N-Ter-Ser-Cys-Val-Gly- Gln-Tyr-Gly-Arg-Cys-Arg-Ser-
·Ala-Tyr-Gln-Asp-Cys-Cys-Asp-Gly- Tyr-Tyr-Cys-Asn-Cys-
Ser-Gln-Pro-Pro-Tyr-Cys-Leu-Cys-Arg-Asn- Asn-Asn

(Curtatoxin III)
 and the salts thereof.

5. A process for isolating Curtatoxin III according to Claim 4 of the whole venom
of the spider Hololena curta which comprises applying 0.5 ml of whole venom to a Pharmacia Pep RPC HR 16/10 HPLC column at a flow rate of 2.5 ml/min, running a linear gradient to 60% acetonitrile containing 0.05% TFA over 100 minutes at a flow rate of 2.5 ml/min., collecting fractions in 5 ml, 2 minute fractions, and selectively isolating the 27th fraction eluting from the column.

6. A process far isolating Curtatoxin I according to Claim 2 of the whole venom
of the spider Hololena curta which comprises applying 0.5 ml of whole venom to a Pharmacia Pep RPC HR 16/10 HPLC column at a flow rate of 2.5 ml/min, running a linear gradient to 60% acetonitrile containing 0.05% TFA over 100 minutes at a flow rate of 2.5 ml/min., collecting fractions in 5 ml, 2 minute fractions, and selectively isolating the 33rd fraction eluting from the column.

7. A process for isolating Curtatoxin II according to Claim 3 of the whole venom
of the spider Hololena curta which comprises applying 0.5 ml of whole venom to a Pharmacia Pep RPC HR 16/10 HPLC column at a flow rate of 2.5 ml/min, running a linear gradient to 60% acetonitrile containing 0.05% TFA over 100 minutes at a flow rate of 2.5 ml/min., collecting fractions in 5 ml, 2 minute fractions, and selectively isolating the 37th fraction eluting from the column.

8. Use of the polypeptides according to any of Claims 1 to 4 for the preparation of an insecticide.

9. Use of the polypeptides according to any of Claims 1 to 4 for the preparation of a pharmaceutical composition.

10. A composition containing an effective amount of a polypeptide of any of Claims 1 to 4 or a mixture thereof optionally in combination with an inert carrier and/or diluent.

11. The composition of Claim 10 as an insecticidal composition.

12. The composition of Claim 10 as a pharmaceutical composition.

13. The pharmaceutical composition of Claim 12 for the treatment of neurological disorders.

14. The pharmaceutical composition of Claim 13 containing said polypeptide in an effective glutamate antagonist amount.

15. The pharmaceutical composition of Claim 13 containing said polypeptide in an effective calcium channel antagonist amount.

## Claims (Claims for the following Contracting State(s): ES)

1. A process for isolating fractions of the whole venom of the spider Hololena curta which comprises applying 0.5 ml of whole venom to a Pharmacia Pep RPC HR 16/10 HPLC column at a flow rate of 2.5 ml/min, running a linear gradient to 60% acetonitrile containing 0.05% TFA over 100 minutes at a flow rate of 2.5 ml/min., collecting fractions in 5 ml, 2 minute fractions, and selectively isolating the 27th, 33th and 37th fraction eluting from the column.

2. A process according to claim 1 wherein the polypeptide of the formula 
N-Ter-Ser-Cys-Val-Gly-Gln-Tyr-Gly-Arg-Cys-Arg-Ser-Ala-
Tyr-Gln-Asp-Cys-Cys-Asp-Gly-Tyr-Tyr-Cys-Asn-Cys-Ser-Gln-
Pro-Pro-Tyr-Cys-Leu-Cys-Arg-Asn-Asn-Asn

(Curtatoxin III)
 is isolated as fraction 27.

3. A process according to claim 1 wherein the polypeptide of the formula 
N-Ter-Ala-Asp-Cys-Val-Gly-Asp-Gly-Gln-Arg-Cys-Ala-Asp-
Trp-Ala-Gly-Pro-Tyr-Cys-Cys-Ser-Gly-Tyr-Tyr-Cys-Ser-Cys-
Arg-Ser-Met-Pro-Tyr-Cys-Arg-Cys-Arg-Ser-Asp-Ser

(Curtatoxin I)
 is isolated as fraction 33.

4. A process according to claim 1 wherein the polypeptide of the formula 
N-Ter-Ala-Asp-Cys-Val-Gly-Asp-Gly-Gln-Lys-Cys-Ala-Asp-
Trp-Phe-Gly-Pro-Tyr-Cys-Cys-Ser-Gly-Tyr-Tyr-Cys-Ser-Cys-
Arg-Ser-Met-Pro-Tyr-Cys-Arg-Cys-Arg-Ser-Asp-Ser

(Curtatoxin II)
 is isolated as fraction 37.

5. Use of the polypeptides according to any of Claims 2 to 4 as insecticides optionally in combination with an inert carrier and/or diluent.

6. Insecticides containing a polypeptide according to any of Claims 2 to 4 or mixtures thereof.

## Claims (Claims for the following Contracting State(s): GR)

1. A polypeptide of the formula 
N-Ter-Ala-Asp-Cys-Val-Gly-Asp-Gly-Gln-X-Cys-Ala-Asp-
Trp-Y-Gly-Pro-Tyr-Cys-Cys-Ser-Gly-Tyr-Tyr-Cys-Ser-Cys-
Arg-Ser-Met-Pro-Tyr-Cys-Arg-Cys-Arg-Ser-Asp-Ser
(Formula I)
 wherein X is Arg or Lys; and Y is Ala or Phe, with the proviso that when X is Arg then Y must be Ala, and when X is Lys then Y must be Phe,
and the salts thereof.

2. A polypeptide according to Claim 1 of the formula

N-Ter-Ala-Asp-Cys-Val-Gly-Asp-Gly-Gln-Arg-Cys-Ala-Asp-
Trp-Ala-Gly-Pro-Tyr-Cys-Cys-Ser-Gly-Tyr-Tyr-Cys-Ser-
Cys-Arg-Ser-Met-Pro-Tyr-Cys-Arg-Cys-Arg-Ser-Asp-Ser.
(Curtatoxin I)

3. A polypeptide according to Claim 1 of the formula

N-Ter-Ala-Asp-Cys-Val-Gly-Asp-Gly-Gln-Lys-Cys-Ala-Asp-
Trp-Phe-Gly-Pro-Tyr-Cys-Cys-Ser-Gly-Tyr-Tyr-Cys-Ser-
Cys-Arg-Ser-Met-Pro-Tyr-Cys-Arg-Cys-Arg-Ser-Asp-Ser.
(Curtatoxin II)

4. A polypeptide of the formula 
N-Ter-Ser-Cys-Val-Gly- Gln-Tyr-Gly-Arg-Cys-Arg-Ser-
-Ala-Tyr-Gln-Asp-Cys-Cys-Asp-Gly- Tyr-Tyr-Cys-Asn-Cys-
Ser-Gln-Pro-Pro-Tyr-Cys-Leu-Cys-Arg-Asn- Asn-Asn

(Curtatoxin III)
 and the salts thereof.

5. A process for isolating Curtatoxin III according to Claim 4 of the whole venom
of the spider Hololena curta which comprises applying 0.5 ml of whole venom to a Pharmacia Pep RPC HR 16/10 HPLC column at a flow rate of 2.5 ml/min, running a linear gradient to 60% acetonitrile containing 0.05% TFA over 100 minutes at a flow rate of 2.5 ml/min., collecting fractions in 5 ml, 2 minute fractions, and selectively isolating the 27th fraction eluting from the column.

6. A process for isolating Curtatoxin I according to Claim 2 of the whole venom
of the spider Hololena curta which comprises applying 0.5 ml of whole venom to a Pharmacia Pep RPC HR 16/10 HPLC column at a flow rate of 2.5 ml/min, running a linear gradient to 60% acetonitrile containing 0.05% TFA over 100 minutes at a flow rate of 2.5 ml/min., collecting fractions in 5 ml, 2 minute fractions, and selectively isolating the 33rd fraction eluting from the column.

7. A process for isolating Curtatoxin II according to Claim 3 of the whole venom
of the spider Hololena curta which comprises applying 0.5 ml of whole venom to a Pharmacia Pep RPC HR 16/10 HPLC column at a flow rate of 2.5 ml/min, running a linear gradient to 60% acetonitrile containing 0.05% TFA over 100 minutes at a flow rate of 2.5 ml/min., collecting fractions in 5 ml, 2 minute fractions, and selectively isolating the 37th fraction eluting from the column.

8. Use of the polypeptides according to any of Claims 1 to 4 for the preparation of an insecticide.

9. A composition containing an effective amount of a polypeptide of any of Claims 1 to 4 or a mixture thereof optionally in combination with an inert carrier and/or diluent.

10. The composition of Claim 10 as an insecticidal composition.

## Patentansprüche (Patentansprüche für folgende(n) Vertragsstaat(en): AT, BE, CH, DE, FR, GB, IT, LI, LU, NL, SE)

1. Polypeptid der Formel 
N-Ter-Ala-Asp-Cys-Val-Gly-Asp-Gly-Gln-X-Cys-Ala-Asp-
Trp-Y-Gly-Pro-Tyr-Cys-Cys-Ser-Gly-Tyr-Tyr-Cys-Ser-Cys-
Arg-Ser-Met-Pro-Tyr-Cys-Arg-Cys-Arg-Ser-Asp-Ser
(Formel I)
 in der X Arg oder Lys bedeutet und Y Ala oder Phe darstellt, mit der Maßgabe, daß wenn X Arg ist, Y Ala sein muß und wenn X Lys ist, Y Phe sein muß, und die Salze davon.

2. Polypeptid nach Anspruch 1 der Formel

N-Ter-Ala-Asp-Cys-Val-Gly-Asp-Gly-Gln-Arg-Cys-Ala-Asp-
Trp-Ala-Gly-Pro-Tyr-Cys-Cys-Ser-Gly-Tyr-Tyr-Cys-Ser-
Cys-Arg-Ser-Met-Pro-Tyr-Cys-Arg-Cys-Arg-Ser-Asp-Ser.
(Curtatoxin I)

3. Polypeptid nach Anspruch 1 der Formel

N-Ter-Ala-Asp-Cys-Val-Gly-Asp—Gly—Gln-Lys-Cys-Ala-Asp-
Trp-Phe-Gly-Pro-Tyr-Cys-Cys-Ser-Gly-Tyr-Tyr-Cys-Ser-
Cys-Arg-Ser-Met-Pro-Tyr-Cys-Arg-Cys-Arg-Ser-Asp-Ser.
(Curtatoxin II)

4. Polypeptid der Formel 
N-Ter-Ser-Cys-Val-Gly- Gln-Tyr-Gly-Arg-Cys-Arg-Ser-
Ala-Tyr-Gln-Asp-Cys-Cys-Asp-Gly- Tyr-Tyr-Cys-Asn-Cys-
Ser-Gln-Pro-Pro-Tyr-Cys-Leu-Cys-Arg-Asn- Asn-Asn
(Curtatoxin III)
 und die Salze davon.

5. Verfahren zur Isolierung von Curtatoxin III gemäß Anspruch 4 aus dem Gesamtgift der Spinne Hololena curta, umfassend das Aufbringen von 0,5 ml des Gesamtgifts auf eine Pharmacia Pep RPC HR 16/10-HPLC-Säule bei einer Fließgeschwindigkeit von 2,5 ml/min, das Anlegen eines linearen Gradienten bis 60 % Acetonitril, enthaltend 0,05 % TFA, über einen Zeitraum von 100 Minuten bei einer Fließgeschwindigkeit von 2,5 ml/min, das Sammeln von Fraktionen in 5 ml-(2 Minuten)-Fraktionen, und das selektive Isolieren der 27ten Fraktion, die von der Säule eluiert wird.

6. Verfahren zur Isolierung von Curtatoxin I gemäß Anspruch 2 aus dem Gesamtgift der Spinne Hololena curta, umfassend das Aufbringen von 0,5 ml des Gesamtgifts auf eine Pharmacia Pep RPC HR 16/10-HPLC-Säule bei einer Fließgeschwindigkeit von 2,5 ml/min, das Anlegen eines linearen Gradienten bis 60 % Acetonitril, enthaltend 0,05 % TFA, über einen Zeitraum von 100 Minuten bei einer Fließgeschwindigkeit von 2,5 ml/min, das Sammeln von 5 ml (2 Minuten)-Fraktionen, und das selektive Isolieren der 33ten Fraktion, die von der Säule eluiert wird.

7. Verfahren zur Isolierung von Curtatoxin II gemäß Anspruch 3 aus dem Gesamtgift der Spinne Hololena curta, umfassend das Aufbringen von 0,5 ml des Gesamtgifts auf eine Pharmacia Pep RPC HR 16/10 HPLC-Säule bei einer Fließgeschwindigkeit von 2,5 ml/min, das Anlegen eines linearen Gradienten bis 60 % Acetonitril, enthaltend 0,05 % TFA, über einen Zeitraum von 100 Minuten bei einer Fließgeschwindigkeit von 2,5 ml/min, das Sammeln von 5 ml (2 Minuten)-Fraktionen, und das selektive Isolieren der 37ten Fraktion, die von der Säule eluiert wird.

8. Verwendung der Polypeptide gemäß einem der Ansprüche 1 bis 4 für die Herstellung eines Insektizids.

9. Verwendung der Polypeptide gemäß einem der Ansprüche 1 bis 4 für die Herstellung eines Arzneimittels.

10. Mittel, enthaltend eine wirksame Menge eines Polypeptids nach einem der Ansprüche 1 bis 4 oder ein Gemisch davon, gegebenenfalls in Kombination mit einem inerten Träger und/oder Verdünnungsmittel.

11. Mittel nach Anspruch 10 als Insektizid.

12. Mittel nach Anspruch 10 als Arzneimittel.

13. Arzneimittel nach Anspruch 12 für die Behandlung von neurologischen Störungen.

14. Arzneimittel nach Anspruch 13, enthaltend das Polypeptid in einer als Glutamat-Antagonist wirksamen Menge.

15. Arzneimittel nach Anspruch 13, enthaltend das Polypeptid in einer als Calciumkanal-Antagonist wirksamen Menge.

## Patentansprüche (Patentansprüche für folgende(n) Vertragsstaat(en): ES)

1. Verfahren zur Isolierung von Fraktionen aus dem Gesamtgift der Spinne Hololena curta, umfassend das Aufbringen von 0,5 ml des Gesamtgifts auf eine Pharmacia Pep RPC HR 16/10-HPLC-Säule bei einer Fließgeschwindigkeit von 2,5 ml/min, das Anlegen eines linearen Gradienten bis 60 % Acetonitril, enthaltend 0,05 % TFA, über einen Zeitraum von 100 Minuten bei einer Fließgeschwindigkeit von 2,5 ml/min, das Sammeln von Fraktionen in 5 ml (2 Minuten)-Fraktionen, und das selektive Isolieren der 27ten, 33ten und 37ten Fraktion, die von der Säule eluiert werden.

2. Verfahren nach Anspruch 1, wobei das Polypeptid der Formel 
N-Ter-Ser-Cys-Val-Gly-Gln-Tyr-Gly-Arg-Cys-Arg-Ser-Ala-
Tyr-Gln-Asp-Cys-Cys-Asp-Gly-Tyr-Tyr-Cys-Asn-Cys-Ser-Gln-
Pro-Pro-Tyr-Cys-Leu-Cys-Arg-Asn-Asn-Asn

(Curtatoxin III)
 aus Fraktion 27 isoliert wird.

3. Verfahren nach Anspruch 1, wobei das Polypeptid der Formel 
N-Ter-Ala-Asp-Cys-Val-Gly-Asp-Gly-Gln-Arg-Cys-Ala-Asp-
Trp-Ala-Gly-Pro-Tyr-Cys-Cys-Ser-Gly-Tyr-Tyr-Cys-Ser-Cys-
Arg-Ser-Met-Pro-Tyr-Cys-Arg-Cys-Arg-Ser-Asp-Ser

(Curtatoxin I)
 aus Fraktion 33 isoliert wird.

4. Verfahren nach Anspruch 1, wobei das Polypeptid der Formel 
N-Ter-Ala-Asp-Cys-Val-Gly-Asp-Gly-Gln-Lys-Cys-Ala-Asp-
Trp-Phe-Gly-Pro-Tyr-Cys-Cys-Ser-Gly-Tyr-Tyr-Cys-Ser-Cys-
Arg-Ser-Met-Pro-Tyr-Cys-Arg-Cys-Arg-Ser-Asp-Ser

(Curtatoxin II)
 aus Fraktion 37 isoliert wird.

5. Verwendung der Polypeptide gemäß einem der Ansprüche 2 bis 4 als Insektizid, gegebenenfalls in Kombination mit einem inerten Träger und/oder Verdünnungsmittel.

6. Insektizide, enthaltend ein Polypeptid nach einem der Ansprüche 2 bis 4 oder Gemische davon.

## Patentansprüche (Patentansprüche für folgende(n) Vertragsstaat(en): GR)

1. Polypeptid der Formel 
N-Ter-Ala-Asp-Cys-Val-Gly-Asp-Gly-Gln-X-Cys-Ala-Asp-
Trp-Y-Gly-Pro-Tyr-Cys-Cys-Ser-Gly-Tyr-Tyr-Cys-Ser-Cys-
Arg-Ser-Met-Pro-Tyr-Cys-Arg-Cys-Arg-Ser-Asp-Ser
(Formel I)
 in der X Arg oder Lys bedeutet und Y Ala oder Phe darstellt, mit der Maßgabe, daß wenn X Arg ist, Y Ala sein muß und wenn X Lys ist, Y Phe sein muß, und die Salze davon.

2. Polypeptid nach Anspruch 1 der Formel

N-Ter-Ala-Asp-Cys-Val-Gly-Asp-Gly-Gln-Arg-Cys-Ala-Asp-
Trp-Ala-Gly-Pro-Tyr-Cys-Cys-Ser-Gly-Tyr-Tyr-Cys-Ser-
Cys-Arg-Ser-Met-Pro-Tyr-Cys-Arg-Cys-Arg-Ser-Asp-Ser.
(Curtatoxin I)

3. Polypeptid nach Anspruch 1 der Formel

N-Ter-Ala-Asp-Cys-Val-Gly-Asp-Gly-Gln-Lys-Cys-Ala-Asp-
Trp-Phe-Gly-Pro-Tyr-Cys-Cys-Ser-Gly-Tyr-Tyr-Cys-Ser-
Cys-Arg-Ser-Met-Pro-Tyr-Cys-Arg-Cys-Arg-Ser-Asp-Ser.
(Curtatoxin II)

4. Polypeptid der Formel 
N-Ter-Ser-Cys-Val-Gly- Gln-Tyr-Gly-Arg-Cys-Arg-Ser-
Ala-Tyr-Gln-Asp-Cys-Cys-Asp-Gly- Tyr-Tyr-Cys-Asn-Cys-
Ser-Gln-Pro-Pro-Tyr-Cys-Leu-Cys-Arg-Asn- Asn-Asn

(Curtatoxin III)
 und die Salze davon.

5. Verfahren zur Isolierung von Curtatoxin III gemäß Anspruch 4 aus dem Gesamtgift der Spinne Hololena curta, umfassend das Aufbringen von 0,5 ml des Gesamtgifts auf eine Pharmacia Pep RPC HR 16/10-HPLC-Säule bei einer Fließgeschwindigkeit von 2,5 ml/min, das Anlegen eines linearen Gradienten bis 60 % Acetonitril, enthaltend 0,05 % TFA, über einen Zeitraum von 100 Minuten bei einer Fließgeschwindigkeit von 2,5 ml/min, das Sammeln von Fraktionen in 5 ml-(2 Minuten)-Fraktionen, und das selektive Isolieren der 27ten Fraktion, die von der Säule eluiert wird.

6. Verfahren zur Isolierung von Curtatoxin I gemäß Anspruch 2 aus dem Gesamtgift der Spinne Hololena curta, umfassend das Aufbringen von 0,5 ml des Gesamtgifts auf eine Pharmacia Pep RPC HR 16/10-HPLC-Säule bei einer Fließgeschwindigkeit von 2,5 ml/min, das Anlegen eines linearen Gradienten bis 60 % Acetonitril, enthaltend 0,05 % TFA, über einen Zeitraum von 100 Minuten bei einer Fließgeschwindigkeit von 2,5 ml/min, das Sammeln von 5 ml (2 Minuten)-Fraktionen, und das selektive Isolieren der 33ten Fraktion, die von der Säule eluiert wird.

7. Verfahren zur Isolierung von Curtatoxin II gemäß Anspruch 3 aus dem Gesamtgift der Spinne Hololena curta, umfassend das Aufbringen von 0,5 ml des Gesamtgifts auf eine Pharmacia Pep RPC HR 16/10 HPLC-Säule bei einer Fließgeschwindigkeit von 2,5 ml/min, das Anlegen eines linearen Gradienten bis 60 % Acetonitril, enthaltend 0,05 % TFA, über einen Zeitraum von 100 Minuten bei einer Fließgeschwindigkeit von 2,5 ml/min, das Sammeln von 5 ml (2 Minuten)-Fraktionen, und das selektive Isolieren der 37ten Fraktion, die von der Säule eluiert wird.

8. Verwendung der Polypeptide gemäß einem der Ansprüche 1 bis 4 für die Herstellung eines Insektizids.

9. Mittel, enthaltend eine wirksame Menge eines Polypeptids nach einem der Ansprüche 1 bis 4 oder ein Gemisch davon, gegebenenfalls in Kombination mit einem inerten Träger und/oder Verdünnungsmittel.

10. Mittel nach Anspruch 9 als Insektizid.

## Revendications (Revendications pour l'(les) Etat(s) contractant(s) suivant(s): AT, BE, CH, DE, FR, GB, IT, LI, LU, NL, SE)

1. Polypeptide de formule 
N-Ter-Ala-Asp-Cys-Val-Gly-Asp-Gly-Gln-X-Cys-Ala-Asp-Trp-Y-Gly-Pro-
Tyr-Cys-Cys-Ser-Gly-Tyr-Tyr-Cys-Ser-Cys-Arg-Ser-Met-Pro-Tyr-Cys-Arg-
Cys-Arg-Ser-Asp-Ser
(Formule I)
 où X est Arg ou Lys ; et Y est Ala ou Phe, sous réserve que si X est Arg, alors Y doit être Ala, et si X est Lys alors Y doit être Phe,
et ses sels.

2. Polypeptide selon la revendication 1, de formule

N-Ter-Ala-Asp-Cys-Val-Gly-Asp-Gly-Gln-Arg-Cys-Ala-Asp-Trp-Ala-Gly-
Pro-Tyr-Cys-Cys-Ser-Gly-Tyr-Tyr-Cys-Ser-Cys-Arg-Ser-Met-Pro-Tyr-Cys-
Arg-Cys-Arg-Ser-Asp-Ser
(Curtatoxine I)

3. Polypeptide selon la revendication 1, de formule

N-Ter-Ala-Asp-Cys-Val-Gly-Asp-Gly-Gln-Lys-Cys-Ala-Asp-Trp-Phe-Gly-
Pro-Tyr-Cys-Cys-Ser-Gly-Tyr-Tyr-Cys-Ser-Cys-Arg-Ser-Met-Pro-Tyr-Cys-
Arg-Cys-Arg-Ser-Asp-Ser
(Curtatoxine II)

4. Polypeptide selon la revendication 1, de formule 
N-Ter-Ser-Cys-Val-Gly-Gln-Tyr-Gly-Arg-Cys-Arg-Ser-Ala-Tyr-Gln-Asp-
Cys-Cys-Asp-Gly-Tyr-Tyr-Cys-Asn-Cys-Ser-Gln-Pro-Pro-Tyr-Cys-Leu-Cys-
Arg-Asn-Asn-Asn
(Curtatoxine III)
 et ses sels.

5. Procédé pour isoler du venin total de l'araignée Hololena curta la Curtatoxine III selon la revendication 4, procédé qui comprend l'application de 0,5 ml de venin total sur une colonne HPLC Pharmacia Pep RPC HR 16/10 à un débit de 2,5 ml/mn, le passage d'un gradient linéaire allant jusqu'à 60 % d'acétonitrile contenant 0,05 % de TFA en 100 minutes à un débit de 2,5 ml/mn, le recueil de fractions par fractions de 5 ml, 2 minutes et l'isolement sélectif de la 27ème fraction éluant de la colonne.

6. Procédé pour isoler du venin total de l'araignée Hololena curta la curtatoxine I selon la revendication 2, procédé qui comprend l'application de 0,5 ml de venin total sur une colonne HPLC Pharmacia Pep RPC HR 16/10 à un débit de 2,5 ml/mn, le passage d'un gradient linéaire allant jusqu'à 60 % d'acétonitrile contenant 0,05 % de TFA en 100 minutes à un débit de 2,5 ml/mn, le recueil de fractions par fractions de 5 ml, 2 minutes et l'isolement sélectif de la 33ème fraction éluant de la colonne.

7. Procédé pour isoler du venin total de l'araignée Hololena curta la Curtatoxine II selon la revendication 3, procédé qui comprend l'application de 0,5 ml de venin total sur une colonne HPLC Pharmacia Pep RPC HR 16/10 à un débit de 2,5 ml/mn, le passage d'un gradient linéaire allant jusqu'à 60 % d'acétonitrile contenant 0,05 % de TFA en 100 minutes à un débit de 2,5 ml/mn, le recueil de fractions par fractions de 5 ml, 2 minutes et l'isolement sélectif de la 37ème fraction éluant de la colonne.

8. Utilisation des polypeptides selon une quelconque des revendications 1 à 4 pour la préparation d'un insecticide.

9. Utilisation des polypeptides selon une quelconque des revendications 1 à 4 pour la préparation d'une composition pharmaceutique.

10. Composition contenant une quantité efficace d'un polypeptide de l'une quelconque des revendications 1 à 4 ou d'un mélange de tels polypeptides facultativement en association avec un diluant et/ou un support inerte.

11. Composition de la revendication 10 en tant que composition insecticide.

12. Composition de la revendication 10 en tant que composition pharmaceutique.

13. Composition pharmaceutique de la revendication 12 pour le traitement des troubles neurologiques.

14. Composition pharmaceutique de la revendication 13 contenant ledit polypeptide en quantité efficace comme antagoniste de glutamate.

15. Composition pharmaceutique de la revendication 13 contenant ledit polypeptide en quantité efficace comme antagoniste des canaux calciques.

## Revendications (Revendications pour l'(les) Etat(s) contractant(s) suivant(s): ES)

1. Procédé pour isoler des fractions du venin total de l'araignée Hololena curta qui comprend l'application de 0,5 ml de venin total sur une colonne HPLC Pharmacia Pep RPC HR 16/10 à un débit de 2,5 ml/mn, le passage d'un gradient linéaire allant jusqu'à 60 % d'acétonitrile contenant 0,05 % de TFA en 100 minutes à un débit de 2,5 ml/mn, le recueil de fractions par fractions de 5 ml, 2 minutes et l'isolement sélectif des 27ème, 33ème, et 37ème fractions éluant de la colonne.

2. Procédé selon la revendication 1 dans lequel le polypeptide de formule 
N-Ter-Ser-Cys-Val-Gly-Gln-Tyr-Gly-Arg-Cys-Arg-Ser-Ala-Tyr-Gln-Asp-
Cys-Cys-Asp-Gly-Tyr-Tyr-Cys-Asn-Cys-Ser-Gln-Pro-Pro-Tyr-Cys-Leu-Cys-
Arg-Asn-Asn-Asn
(Curtatoxine III)
 est isolé en tant que fraction 27.

3. Procédé selon la revendication 1 dans lequel le polypeptide de formule 
N-Ter-Ala-Asp-Cys-Val-Gly-Asp-Gly-Gln-Arg-Cys-Ala-Asp-Trp-Ala-Gly-
Pro-Tyr-Cys-Cys-Ser-Gly-Tyr-Tyr-Cys-Ser-Cys-Arg-Ser-Met-Pro-Tyr-Cys-
Arg-Cys-Arg-Ser-Asp-Ser
(Curtatoxine I)
 est isolé en tant que fraction 33

4. Procédé selon la revendication 1 dans lequel le polypeptide de formule 
N-Ter-Ala-Asp-Cys-Val-Gly-Asp-Gly-Gln-Lys-Cys-Ala-Asp-Trp-Phe-Gly-
Pro-Tyr-Cys-Cys-Ser-Gly-Tyr-Tyr-Cys-Ser-Cys-Arg-Ser-Met-Pro-Tyr-Cys-
Arg-Cys-Arg-Ser-Asp-Ser
(Curtatoxine II)
 est isolé sous forme de fraction 37.

5. Utilisation des polypeptides selon une quelconque des revendications 2 à 4 en tant qu'insecticides facultativement en association avec un diluant et/ou un support inerte.

6. Insecticides contenant un polypeptide selon une quelconque des revendications 2 à 4 ou un mélange de tels polypeptides.

## Revendications (Revendications pour l'(les) Etat(s) contractant(s) suivant(s): GR)

1. Polypeptide de formule 
N-Ter-Ala-Asp-Cys-Val-Gly-Asp-Gly-Gln-X-Cys-Ala-Asp-Trp-Y-Gly-Pro-
Tyr-Cys-Cys-Ser-Gly-Tyr-Tyr-Cys-Ser-Cys-Arg-Ser-Met-Pro-Tyr-Cys-Arg-
Cys-Arg-Ser-Asp-Ser
(Formule I)
 où X est Arg ou Lys ; et Y est Ala ou Phe, sous réserve que si X est Arg, alors Y doit être Ala, et si X est Lys alors Y doit être Phe,
et ses sels.

2. Polypeptide selon la revendication 1 de formule

N-Ter-Ala-Asp-Cys-Val-Gly-Asp-Gly-Gln-Arg-Cys-Ala-Asp-Trp-Ala-Gly-
Pro-Tyr-Cys-Cys-Ser-Gly-Tyr-Tyr-Cys-Ser-Cys-Arg-Ser-Met-Pro-Tyr-Cys-
Arg-Cys-Arg-Ser-Asp-Ser
(Curtatoxine I)

3. Polypeptide selon la revendication 1 de formule

N-Ter-Ala-Asp-Cys-Val-Gly-Asp-Gly-Gln-Lys-Cys-Ala-Asp-Trp-Phe-Gly-
Pro-Tyr-Cys-Cys-Ser-Gly-Tyr-Tyr-Cys-Ser-Cys-Arg-Ser-Met-Pro-Tyr-Cys-
Arg-Cys-Arg-Ser-Asp-Ser
(Curtatoxine II)

4. Polypeptide selon la revendication 1 de formule 
N-Ter-Ser-Cys-Val-Gly-Gln-Tyr-Gly-Arg-Cys-Arg-Ser-Ala-Tyr-Gln-Asp-
Cys-Cys-Asp-Gly-Tyr-Tyr-Cys-Asn-Cys-Ser-Gln-Pro-Pro-Tyr-Cys-Leu-Cys-
Arg-Asn-Asn-Asn
(Curtatoxine III)
 et ses sels.

5. Procédé pour isoler du venin total de l'araignée Hololena curta la Curtatoxine III selon la revendication 4, procédé qui comprend l'application de 0,5 ml de venin total sur une colonne HPLC Pharmacia Pep RPC HR 16/10 à un débit de 2,5 ml/mn, le passage d'un gradient linéaire allant jusqu'à 60 % d'acétonitrile contenant 0,05 % de TFA en 100 minutes à un débit de 2,5 ml/mn, le recueil de fractions par fractions de 5 ml, 2 minutes et l'isolement sélectif de la 27ème fraction éluant de la colonne.

6. Procédé pour isoler du venin total de l'araignée Hololena curta la curtatoxme I selon la revendication 2, procédé qui comprend l'application de 0,5 ml de venin total sur une colonne HPLC Pharmacia Pep RPC HR 16/10 à un débit de 2,5 ml/mn, le passage d'un gradient linéaire allant jusqu'à 60 % d'acétonitrile contenant 0,05 % de TFA en 100 minutes à un débit de 2,5 ml/mn, le recueil de fractions par fractions de 5 ml, 2 minutes et l'isolement sélectif de la 33ème fraction éluant de la colonne.

7. Procédé pour isoler du venin total de l'araignée Hololena curta la Curtatoxine II selon la revendication 3, procédé qui comprend l'application de 0,5 ml de venin total sur une colonne HPLC Pharmacia Pep RPC HR 16/10 à un débit de 2,5 ml/mn, le passage d'un gradient linéaire allant jusqu'à 60 % d'acétonitrile contenant 0,05 % de TFA en 100 minutes à un débit de 2,5 ml/mn, le recueil de fractions par fractions de 5 ml, 2 minutes et l'isolement sélectif de la 37ème fraction éluant de la colonne.

8. Utilisation des polypeptides selon une quelconque des revendications 1 à 4 pour la préparation d'un insecticide.

9. Composition contenant une quantité efficace d'un polypeptide de l'une quelconque des revendications 1 à 4 ou d'un mélange de tels polypeptides facultativement en association avec un diluant et/ou un support inerte.

10. Composition de la revendication 10 en tant que composition insecticide.
